# EUROPEAN PATENT SPECIFICATION

(11) **EP 1 339 414 B1**
(45) Date of publication and mention of the grant of the patent: **06.01.2010**
(21) Application number: 01993437.1
(22) Date of filing: 08.11.2001
(51) Int. Cl.: A61K 33/40, A61K 31/415, A61K 47/32, A61P 27/02

(54) **L-HISTIDINE IN OPHTHALMIC SOLUTIONS**
L-HISTIDIN IN OPHTHALMOLOGISCHEN LÖSUNGEN
L-HISTIDINE DANS DES SOLUTIONS OPHTALMIQUES

(30) Priority: 08.11.2000 US 246689 P; 08.11.2000 US 246707 P; 08.11.2000 US 246708 P; 08.11.2000 US 246709 P
(43) Date of publication of application: 03.09.2003
(73) Proprietor: FXS Ventures, LLC, Salem, NH 03079 (US)
(72) Inventor: SMITH, Francis, Xavier, Salem, NH 03079 (US)
(74) Representative: Wagner, Karl H.
(86) International application number: PCT/US2001/046762
(87) International publication number: WO 2002/038077

(56) References cited:
- EP-A- 0 923 950
- WO-A-92/11876
- US-A- 5 925 317
- US-A- 5 952 320
- US-A- 6 117 869

## Description

The present invention relates to the field of ophthalmic solutions used to treat eyes, store contact lenses, or condition medical devices used in the eye. Such solutions are well known and widely employed with numerous products available commercially. There are several types of solutions within the field depending upon specific use. For instance, there are specific solutions for disinfecting contact lenses, solutions for cleaning contact lenses, solutions for treating the surface of contact lenses, solutions for rinsing lenses, solutions for wetting eyes, etc.

While each of these lenses are formulated specifically for an intended application, each solution is formulated or handled so that it will remain free of sources of infection to they eye. Numerous approaches to this problem have been employed, from methods that call for sterilization of the solution and packaging of the solution in a container that will not allow contamination. Use of specific preservative agents employed in concentrations sufficient to prevent microbial increase have been employed. Oxidative agents have been used as well as methods of irradiation. In the cases where chemical agents have been employed, there has been a tendency to employ one preservative agent in the formulation. It has been found that use of two or more specific agents in combination surprisingly provide greater efficacy in preserving solutions than state of the art single preservative systems and in particular the use of the combination of a cationic polymeric preservative, hydrogen peroxide and L-histidine provide increased preservative efficacy against fungal contamination.

This surprising effect is achievable with the further use of certain, but not all, contact lens solution agents. In particular, certain tonicity agents when employed decrease the preservative efficacy of the invention and should not be employed.

As described in U.S. Pat. No. 4.029.817, hydrophilic plastic materials are used in making soft contact lenses. U.S. Pat. No. 3,503,393 to Seiderman and U.S. Pat. No. 2,976,576 to Wichterle describe processes for producing hydrophilic polymers of polyhydroxyethylmethacrylate in aqueous reaction media having a sparingly cross-linked polymeric hydrogel structure and being elastic, soft, transparent hydrogels. Other soft contact lenses are made of silicone and other suitable materials.

Hydrophilic lenses are particularly useful in opthalmology due to their ability to absorb water and swell to a soft mass of good mechanical strength, and due to their transparency with the ability to retain shape and dimensions when equilibrated in ocular fluid and in storage fluids when removed from the eye.

One problem with soft contact lenses, however, is their sterilization and cleaning. The property of hydrophilic soft lenses which allows them to absorb large amounts of water also allows preservatives which might otherwise be used for cleaning and sterilization to be absorbed and later released onto the eye. The release, furtheremore, may be much slower than the intake, thereby allowing preservatives to build up in the lenses. This can have the harmful result of damaging or staining contact lenses or harming the sensitive tissues of the conjunctivae or cornea.

As stated by R. E. Phares in U.S. Pat. No. 3,689,673, sterilization of hydrophilic soft contact lenses may be carried out by soaking in an aqueous solution containing approximately 0.001-0.01% chlorhexidine for a time sufficient to sterilize the lens.

Various related methods are disclosed in other U.S. patents. U.S. Pat. No. 3,591,329 discloses the use of a cationic resin exchange material impregnated with active metallic silver. U.S. Pat. No. 3,755,561 teaches using an aqueous solution of polyvinyl pyrrolidone, a polyalkylene glycol and thimerosal. U.S. Pat. No. 3,873,696 discloses using a combination of potassium peroxymonosulfate in the presence of sodium chloride. In U.S. Pat. No. 3,876,768 is described the use of a chlorinated trisodium phosphate material which is similar to hypochlorite. U.S. Pat. No. 3,888,782 relates to the using of chlorhexidine and polyvinyl pyrrolidone. The use of an iodoform solution containing iodine, polyvinyl alcohol and boric acid is disclosed in U.S. Pat. No. 3,911,107. U.S. Pat. No. 3,912,450 proposes using a combination of an alcoholic glutaraldehyde solution containing a surfactant in conjunction with an ultrasonic radiation device.

U.S. Pat. No. 3,888,782 more particularly discloses an aqueous, substantially isotonic cleaning and sterilizing solution for plastic hydrophilic soft contact lenses containing, as active ingredients, chlorhexidine and polyvinylpyrrolidone. The solution is said to be non-toxic to the eye of the wearer of soft contact lenses and in the presence of a suitable amount of water soluble polyhydroxyethylmethacrylate to prevent the build-up of opaque deposits on the surfaces of soft contact lenses.

U.S. Pat. No. 4,029,817 discloses that soft contact lenses may be sterilized by contacting soft lenses with a sterile, aqueous, substantially isotonic solution containing as an active ingredient, an effective amount of a specific quaternary ammonium compound.

United States Patent No. 4758595 teaches a preserving solution comprising a microbicidally or fungicidally effective amount of a biguanide or water-soluble salt thereof, in combination with a buffer system but does not recognize the need to provide a broad spectrum preservative efficacy.
United States Patent No. 4361548 discloses and claims disinfecting and/or preserving solution for contact lenses containing 0.00001 to 0.1 weight percent of a dimethyldiallylammonium chloride homopolymer having a molecular weight from about 10,000 to about 1,000,000, optionally together with up to 0.5 weight percent of ethylenediaminetetraacetic acid or other enhancers and optional buffers and the like, but also does not teach a multiple component preservative.

United States Patent No. 4354952 is directed to a disinfecting and/or preserving solution for contact lenses containing 0.0035 to 0.04 weight percent of an amphoteric surfactant in combination with 0.0005 to 0.01 weight percent of chlorhexidine and 0.002 to 0.025 weight percent of a non-ionic surfactant, optionally together with up to 0.5 weight percent of thimerosal or other enhancers and optional buffers and the like. While a multiple preservative system is disclosed, there is no teaching that the system has more than cumulative advantage.

United States Patent No. 5741817 broadly teaches the use of amino acids, but is specifically addressed to the use of glycine in combination with specific antimicrobial preservatives, not the specific agents employed in the present invention.

United States Patent No. 6022732 teaches that effective hydrogen peroxide based solutions used to disinfect lenses need to be reduced. In particular the patent is directed to Compositions, and methods for using such compositions, which are useful to destroy hydrogen peroxide in a liquid aqueous medium, such as that used to disinfect contact lenses. In one embodiment, the composition comprises a hydrogen peroxide destroying component effective when released in a hydrogen peroxide-containing liquid aqueous medium to destroy or cause the destruction of hydrogen peroxide present in the hydrogen peroxide-containing liquid aqueous medium, and a barrier component acting to substantially prevent the release of the hydrogen peroxide destroying component for a period of time after the composition is initially contacted with the hydrogen peroxide-containing liquid aqueous medium, the barrier component comprising a material selected from the group consisting of water soluble cellulose derivatives and mixtures thereof having a molecular weight of at least about 20,000. The composition results in reduced foam formation relative to a similar composition including a barrier component comprising a similar material having a molecular weight of 10,000 when both the composition and the similar composition are exposed to identical hydrogen peroxide-containing liquid aqueous media to destroy or cause the destruction of the hydrogen peroxide therein.
Similarly directed United States Patent No. 5660862 teaches a composition useful for disinfecting a contact lens comprising a substantially isotonic, aqueous liquid medium containing hydrogen peroxide in an amount effective to disinfect a contact lens contacted with the aqueous liquid medium, and a hydrogen peroxide reducing agent dissolved in the aqueous liquid medium in an amount effective to enhance the antimicrobial activity of the aqueous liquid medium. Preferably, the composition further includes transition metal ions in an amount effective to further enhance the antimicrobial activity of the aqueous liquid medium and is substantially free of peroxidase

United States Patent No. 5854303 teaches a polymeric material incorporating a polyvalent cation chelating agent in an amount effective to inhibit the growth of an ocular pathogen, particularly a protozoan, can be used to produce eye care products such as contact lens cases and containers for containing eye care solutions and contact lenses.
U.S. Pat. No. 4,863,900 teaches that a composition for reducing the transmissability of viral infection from a subject infected therewith which comprises a topically applicable, pharmaceutically acceptable carrier and a viricidally effective amount of a polypeptide of between 24 and 500 aminoacid residues comprising at least 24 residues of L-Histidine. It does not suggest that L-histidine could be used with other bactericidal agents to improve their effect.
U.S. Pat. No. 5741817 demonstrates that glycine enhances the activity of antimicrobial preservatives, and could be used in ophthalmic solutions and are useful as substitutes for EDTA, while U.S. Pat. No. 5,494,937 teaches solutions that contain a combination of glycien with a borate-polyol complex, one or more anionic or nonionic surfactants, and a low molecular weight amino acid (e.g., glycine). This system requires certain anti-bacterial surfactants and no edta. specifically teaches glycine.
U.S. Pat. No. 5925317 further shows the use histidine to neutralize iodine in a two step method to avoid lens discloration. The patent teaches that "histidine is not known to have been previously suggested for use in care regimens for contact lenses, although the oxidation reaction of histidine with an excess of iodine is discussed in a paper by Schutte, L., et al, "The Substitution Reaction of Histidine and Some Other Imidazole Derivatives With Iodine," Tetrahedron, Suppl. 7, pp. 295-306 (1965). One drawback to using an imidazole such as histidine is the formation of an oxidation product that decomposes to a brown degradation product. "
U.S. Pat. No. 6,008,195 returns to the use of polymeric anti-bacterials that have L-histidine as side chain group in the active agent.

### Summary of the Invention

The invention relates to an aqueous ophthalmic solution comprising 0.01 to 1.0 percent by weight L-histidine; 0.0001 to 0.01 percent by weight hydrogen peroxide; 0.1 to 500 parts per million of a cationic polymeric preservative that provides superior preservative efficacy especially as against fungal microbes. These solutions may be employed in various ways including cleaning contact lenses, rinsing lenses while in the eye, storing lenses and in delivering active pharmaceutical agents to the eye.

The invention may also further comprise a surface-active agent chosen from those known in the art, but in particular might be a hydroxy-ethoxylated castor oil.

The solution can be used to deliver a pharmaceutical agent to the eye by providing the agent to the solution and then contacting the eye with the resultant solution. Or the solution can be used to clean, treat or store contact lenses by contacting the solution with the contact lens.

One of the objectives of the invention is to provide an acceptable solution that has a greater kill rate than state of the art solutions.

Another object of the invention is to provide an ophthalmic solution which is effective over a broader range of microbial organisms than state of the art solutions.

### Detailed Description

The invention relates to an aqueous ophthalmic solution comprising 0.01 to 1.0 percent by weight L-histidine; 0.0001 to 0.01 percent by weight hydrogen peroxide; and 0.1 to 500 parts per million of a cationic polymeric preservative that provides superior preservative efficacy, especially as against fungii. These solutions may be employed in various ways including cleaning contact lenses, rinsing lenses while in the eye, storing lenses and in delivering active pharmaceutical agents to the eye. The invention may also further comprise a surface-active agent chosen from those known in the art, but in particular might be a hydroxy-ethoxylated castor oil.
Histidine is a basic amino acid well known in the chemical arts and available from numerous commercial sources. Histidine is known to be used in ophthalmic ointments and the like in very concentrated forms see United States Patent No. 5,811,446.
The cationic polymeric preservative includes polymeric biguanides such as polymeric hexamethylene biguanides (PHMB), and combinations thereof. Such cationic polymeric biguanides, and water-soluble salts thereof, having the following formula: 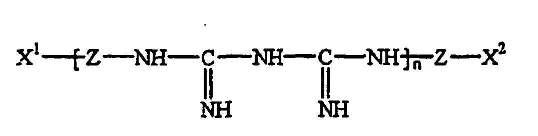 wherein Z is an organic divalent bridging group which may be the same or different throughout the polymer, n is on average at least 3, preferably on average 5 to 20, and X¹ and X² are

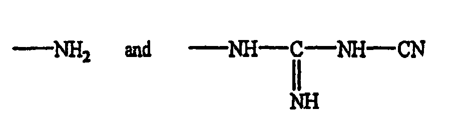

One preferred group of water-soluble polymeric biguanides will have number average molecular weights of at least 1,000 and more preferably will have number average molecular weights from 1,000 to 50,000. Suitable water-soluble salts of the free bases include, but are not limited to hydrochloride, borate, acetate, gluconate, sulfonate, tartrate and citrate salts.

The above-disclosed biguanides and methods of preparation are described in the literature. For example, U.S. Pat. No. 3,428,576 describes the preparation of polymeric biguanides from a diamine and salts thereof and a diamine salt of dicyanimide.

Most preferred are the polymeric hexamethylene biguanides, commercially available, for example, as the hydrochloride salt from Zeneca (Wilmington, Del.) under the trademark CosmocilTM CQ. Such polymers and water-soluble salts are referred to as polyhexamethylene (PHMB) or polyaminoptopyl biguanide (PAPB). The term polyhexamethylene biguanide, as used herein, is meant to encompass one or more biguanides have the following formula: 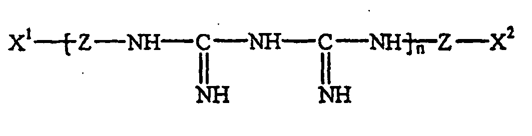 wherein Z, X¹ and X² are as defined above and n is from 1 to 500.

Depending on the manner in which the biguanides are prepared, the predominant compound falling within the above formula may have different X¹ and X² groups or the same groups, with lesser amounts of other compounds within the formula. Such compounds are known and are disclosed in U.S. Pat. No. 4,758,595 and British Patent 1,432,345. Preferably, the water-soluble salts are compounds where n has an average value of 2 to 15, most preferably 3 to 12.

In another embodiment, a polymeric biguanide is used in combination with a bis(biguanide) compound. Polymeric biguanides, in combination with bisbiguanides such as alexidine, are effective in concentrations as low as 0.00001 weight percent (0.1 ppm). It has also been found that the bactericidal activity of the solutions may be enhanced or the spectrum of activity broadened through the use of a combination of such polymeric biguanides with alexidine or similar biguanides.

An optional non-biguanide disinfectant/germicide can be employed as a solution preservative, but it may also function to potentiate, complement or broaden the spectrum of microbiocidal activity of another germicide. This includes microbiocidally effective amounts of germicides which are compatible with and do not precipitate in the solution, in concentrations ranging from about 0.00001 to about 0.5 weight percent, and more preferably, from about 0.0001 to about 0.1 weight percent. Suitable complementary germicidal agents include quaternary ammonium compounds or polymers, thimerosal or other phenylmercuric salts, sorbic acid, alkyl triethanolamines, and mixtures thereof. Representative examples of the quaternary ammonium compounds are compositions comprised of benzalkonium halides or, for example, balanced mixtures of n-alkyl dimethyl benzyl ammonium chlorides. Other examples include polymeric quaternary ammonium salts used in ophthalmic applications such as poly[(dimethyliminio)-2-butene-1,4-diyl chloride], [4-tris(2-hydroxyethyl) ammonio]-2-butenyl-w-[tris(2-hydroxyethyl)ammonio]dichloride (chemical registry number 75345-27-6) generally available as polyquaternium 1 (r) from ONYX Corporation, or those described in U.S. Pat. No. 6,153,568.

Peroxide sources may also be included in the formulations of the present invention and are exemplified by hydrogen peroxide, and such compounds , which provide an effective resultant amount of hydrogen peroxide, such as sodium perborate decahydrate, sodium peroxide, urea peroxide and peracetic acid, an organic peroxy compound .

The pH of the present solutions should be maintained within the range of 5.0 to 8.0, more preferably about 6.0 to 8.0, most preferably about 6.5 to 7.8. Suitable buffers may be added, such as boric acid, sodium borate, potassium citrate, citric acid, sodium bicarbonate, bis-tris propane, TRIS, and various mixed phosphate buffers (including combinations of Na₂HPO₄, NaH₂PO₄ and KH₂PO₄) and mixtures thereof. Borate buffers are preferred, particularly for enhancing the efficacy of PAPB. Generally, buffers will be used in amounts ranging from about 0.05 to 2.5 percent by weight, and preferably, from 0.1 to 1.5 percent.

The solutions of the present invention may further contain other additives including buffers, tonicity agents, demulcents, wetting agents, preservatives, sequestering agents (chelating agents), surface active agents, and enzymes.

Ophthalmologically acceptable chelating agents useful in the present invention include amino carboxylic acid compounds or water-soluble salts thereof, including ethylenediaminetetraacetic acid, nitrilotriacetic acid, diethylenetriamine pentaacetic acid, hydroxyethylethylenediaminetriacetic acid, 1,2-diaminocyclohexanetetraacetic acid, ethylene glycol bis (beta-aminoethyl ether) in N, N, N', N' tetraacetic acid (EGTA), aminodiacetic acid and hydroxyethylamino diacetic acid. These acids can be used in the form of their water soluble salts, particularly their alkali metal salts. Especially preferred chelating agents are the di-, tn- and tetra-sodium salts of ethylenediaminetetraacetic acid (EDTA), most preferably disodium EDTA (Disodium Edetate).

Other chelating agents such as citrates and polyphosphates can also be used in the present invention. The citrates which can be used in the present invention include citric acid and its mono-, di-, and tri-alkaline metal salts. The polyphosphates which can be used include pyrophosphates, triphosphates, tetraphosphates, trimetaphosphates, tetrametaphosphates, as well as more highly condensed phosphates in the form of the neutral or acidic alkali metal salts such as the sodium and potassium salts as well as the ammonium salt. The solutions of the invention are compatible with both rigid gas permeable and hydrophilic contact lenses and other ophthalmic devices and instruments during storage, cleaning, wetting, soaking, rinsing and disinfection.

A typical aqueous solution of the present invention may contain additional ingredients which would not affect the basic and novel characteristics of the active ingredients described earlier, such as tonicity agents, surfactants and viscosity inducing agents, which may aid in either the lens cleaning or in providing lubrication to the eye. Suitable tonicity agents include sodium chloride, potassium chloride, glycerol or mixtures thereof The tonicity of the solution is typically adjusted to approximately 240-310 milliosmoles per kilogram solution (mOsm/kg) to render the solution compatible with ocular tissue and with hydrophilic contact lenses. In one embodiment, the solution contains 0.01 to 0.35 weight percent sodium chloride.

The solutions employed in the present invention may also include surfactants such as a polyoxyethylene-polyoxypropylene nonionic surfactant which, for example, can be selected from the group of commercially available surfactants having the name poloxamine or poloxamer, as adopted by The CTFA International Cosmetic Ingredient Dictionary. The poloxamine surfactants consist of a poly(oxypropylene)-poly(oxyethylene) adduct of ethylene diamine having a molecular weight from about 7,500 to about 27,000 wherein at least 40 weight percent of said adduct is poly(oxyethylene), has been found to be particularly advantageous for use in conditioning contact lenses when used in amounts from about 0.01 to about 15 weight percent. Such surfactants are available from BASF Wyandotte Corp., Wyandotte, Mich., under the registered trademark "Tetronic". The poloxamers are an analogous series of surfactants and are polyoxyethylene, polyoxypropylene block polymers available from BASF Wyandotte Corp., Parsippany, N.J. 07054 under the trademark "Pluronic".

The HLB of a surfactant is known to be a factor in determining the emulsification characteristics of a nonionic surfactant. In general, surfactants with lower HLB values are more lipophilic, while surfactants with higher HLB values are more hydrophilic. The HLB values of various poloxamines and poloxamers are provided by BASF Wyandotte Corp., Wyandotte, Mich. Preferably, the HLB of the surfactant in the present invention is at least 18, more preferably 18 to 32, based on values reported by BASF.

Additional compatible surfactants that are known to be useful in contact wetting or rewetting solutions can be used in the solutions of this invention. The surfactant should be soluble in the lens care solution and non-irritating to eye tissues. Satisfactory non-ionic surfactants include polyethylene glycol esters of fatty acids, e.g. coconut, polysorbate, polyoxyethylene or polyoxypropylene ethers of higher alkanes (C₁₂ - C₁₈). Examples of the preferred class include polysorbate 20 (available from ICI Americas Inc., Wilmington, Del. 19897 under the trademark Tween ® 20), polyoxyethylene (23) lauryl ether (Brij ® 35), polyoxyethylene (40) stearate (Myrj ® 52), polyoxye thylene (25) propylene glycol stearate (Atlas ® G 2612). Brij ® 35, Myrj ® 52 and Atlas ® G 2612 are trademarks of, and are commercially available from, ICI Americas Inc., Wilmington, Del. 19897.

Various other surfactants suitable for in the invention can be readily ascertained, in view of the foregoing description, from McCutcheon's Detergents and Emulsifiers, North American Edition, McCutcheon Division, MC Publishing Co., Glen Rock, N.J. 07452 and the CTFA International Cosmetic Ingredient Handbook, Published by The Cosmetic, Toiletry, and Fragrance Association, Washington, D.C. however, the preferred surfactants are commercially available surfactants sold under the trademark Cremaphor RH40® by BASF which are polyoxyethoxylated castor oils.

### Examples

The following examples illustrate how the invention might be practiced in certain particulars.

### Example 1

Formulations were prepared by dissolving L-histidine in water. The pH of the solutions were adjusted to 7.3 with 1N hydrochloric acid. Hydrogen peroxide, Dequest 2010 and polyhexamethylenebiguanide HCl (PHMB) were added to these solutions. The formulations were diluted to volume with water. Each of these solutions were tested for their activity against *C. albicans* (ATCC 10231) following a two hour exposure. The activity is expressed as a log reduction from the initial inoculum. The compositions, concentrations and activity of each of the solutions are summarized in the following table.

| **Log Reduction** | **Preservative** | **Buffer** | **Hydrogen Peroxide** | **Dequest 2010** |
|---|---|---|---|---|
| 1.25 | PHMB 0.0001% | L-histidine 0.2% | none | 0.006% |
| 1.85 | PHMB 0.0001% | L-histdine 0.2% | 0.006% | 0.006% |

The results demonstrate the improved antifungal efficacy of the histidine - hydrogen peroxide combination against *C. albicans.*

### Example 2

Formulations were prepared by dissolving L-histidine in water. The pH of the solutions were adjusted to 7.3 with 1 N hydrochloric acid. Sodium chloride, Hydrogen peroxide, Dequest 2010 and polyhexamethylenebiguanide HCl (PHMB) were added to these solutions. The formulations were diluted to volume with water. Each of these solutions were tested for their activity against *C. albicans* (ATCC 10231) following a two hour exposure. The activity is expressed as a log reduction from the initial inoculum. The compositions, concentrations and activity of each of the solutions are summarized in the following table.

| **Log Reduction** | **Preservative** | **Buffer** | **Sodium Chloride** | **Hydrogen Peroxide** | **Dequest 2010** |
|---|---|---|---|---|---|
| 0.50 | PHMB 0.0001% | L-histdine 0.2% | 0.4% | none | 0.006% |
| 1.08 | PHMB 0.0001% | L-histdine 0.2% | 0.4% | 0.006% | 0.006% |

The results demonstrate the improved antifungal efficacy of the histidine - hydrogen peroxide combination against *C. albicans.*

### Example 3

Formulations were prepared by dissolving L-histidine in water. The pH of the solutions were adjusted to 7.3 with 1 N hydrochloric acid. Glycerin, hydrogen peroxide, Dequest 2010 and polyhexamethylenebiguanide HCl (PHMB) were added to these solutions. The formulations were diluted to volume with water. Each of these solutions were tested for their activity against *C. albicans* (ATCC 10231) following a two hour exposure. The activity is expressed as a log reduction from the initial inoculum. The compositions, concentrations and activity of each of the solutions are summarized in the following table.

| **Log Reduction** | **Preservative** | **Buffer** | **Glycerin** | **Hydrogen Peroxide** | **Dequest 2010** |
|---|---|---|---|---|---|
| 1.60 | PHMB 0.0001 % | L-Histidine 0.2% | none | none | none |
| 2.38 | PHMB 0.0001 % | L-Histidine 0.2% | none | 0.006% | none |
| | | | | | |
| 1.27 | PHMB 0.0001 % | L-Histidine 0.2% | none | none | 0.006% |
| 2.25 | PHMB 0.0001% | L-Histidine 0.2% | none | 0.006% | 0.006% |
| | | | | | |
| 1.08 | PHMB 0.0001 % | L-Histidine 0.2% | none | none | 0.003% |
| 2.04 | PHMB 0.0001 % | L-Histidine 0.2% | none | 0.006% | 0.003% |
| | | | | | |
| 1.57 | PHMB 0.0001% | L-Histidine 0.2% | 0.50% | none | none |
| 2.15 | PHMB 0.0001 % | L-Histidine 0.2% | 0.50% | 0.006% | none |
| | | | | | |
| 1.25 | PHMB 0.0001% | L-Histidine 0.2% | 0.50% | none | 0.006% |
| 2.04 | PHMB 0.0001% | L-Histidine 0.2% | 0.50% | 0.006% | 0.006% |
| | | | | | |
| 1.08 | PHMB 0.0001% | L-Histidine 0.2% | 0.50% | none | 0.003% |
| 1.93 | PHMB 0.0001% | L-Histidine 0.2% | 0.50% | 0.006% | 0.003% |

The results demonstrate the improved antifungal against *C. albicans* in each paired formulation, when 0.006% hydrogen peroxide is added. The data demonstrates that the increased activity is independent of the presence of Dequest 2010.

### Example 4

Formulations were prepared by dissolving L-histidine in water. The pH of the solutions were adjusted to 7.3 with 1 N hydrochloric acid. Hydrogen peroxide, Dequest 2010 and polyhexamethylenebiguanide HCl (PHMB) were added to these solutions. The formulations were diluted to volume with water. Each of these solutions were tested for their activity against *C. albicans* (ATCC 10231) following a two hour exposure. The activity is expressed as a log reduction from the initial inoculum. The compositions, concentrations and activity of each of the solutions are summarized in the following table.

| **Log Reduction** | **Preservative** | **Buffer** | **Hydrogen Peroxide** | **Dequest 2010** |
|---|---|---|---|---|
| 2.01 | PHMB 0.0001 % | Histidine 0.2% | none | none |
| 2.42 | PHMB 0.0001 % | Histidine 0.2% | 0.006% | 0.003% |
| | | | | |
| 0.73 | Marketed Product 1 | | | |
| 1.95 | Marketed Product 2 | | | |

| | | | | |
|---|---|---|---|---|
| * marketed product 1 having the general compositicn: A sterile isotonic aqueous solution containing sodium chloride, polyoxyethylene polyoxypropylene block copolymer, sodium phosphate dibasic, sodium phosphate monobasic, and preserved with edetate disodium dihydrate 0.025% and polyhexanide 0.0001%. ** marketed product 2 having the general composition: A sterile, isotonic solution that contains HYDRANATE (hydroxyalkylphosphonate), boric acid, edetate disodium, poloxamine, sodium borate and sodium chloride; preserved with DYMED (polyaminopropyl biquanide) 0.0001%. | | | | |

The results demonstrate the improved antifungal efficacy of the histidine - hydrogen peroxide combination. The effectiveness was superior to that found in either commercially marketed products.

### Example 5

Formulations were prepared by dissolving L-histidine in water. The pH of the solutions were adjusted to 7.3 with 1 N hydrochloric acid. Cremophor RH40, hydrogen peroxide, Dequest 2010 and polyhexamethylenebiguanide HCl (PHMB) were added to these solutions. The formulations were diluted to volume with water. Each of these solutions were tested for their activity against *C. albicans* (ATCC 10231) following a two hour exposure. The activity is expressed as a log reduction from the initial inoculum. The compositions, concentrations and activity of each of the solutions are summarized in the following table.

| **Log Reduction** | **Preservative** | **Buffer** | **Additive** | **Hydrogen Peroxide** | **Dequest 2010** |
|---|---|---|---|---|---|
| 2.51 | PHMB 0.0001% | L-Histidine 0.2% | Cremophor RH 40 | none | none |
| 3.27 | PHMB 0.0001% | L-Histidine 0.2% | Cremophor RH 40 | 0.006% | 0.003% |

The results demonstrate the improved antifungal efficacy of the histidine - hydrogen peroxide combination against *C. albicans.*

### Example 6

Formulations were prepared by dissolving L-histidine in water. The pH of the solutions were adjusted to 7.3 with 1 N hydrochloric acid. The tonicity agent, hydrogen peroxide, Dequest 2010 and polyhexamethylenebiguanide HCl (PHMB) were added to these solutions. The formulations were diluted to volume with water. Each of these solutions were tested for their activity against *C. albicans* (ATCC 10231) following a two hour exposure. The activity is expressed as a log reduction from the initial inoculum. The compositions, concentrations and activity of each of the solutions are summarized in the following table.

| **Log Reduction** | **Preservative** | **Buffer** | **Tonicity Agent** | **Wetting Agent** | **Hydrogen Peroxide** | **Dequest 2010** |
|---|---|---|---|---|---|---|
| 2.42 | PHMB 0.0001% | L-Histidine 0.2% | none | Cremophor RH 40 | | |
| 3.34 | PHMB 0.0001% | L-Histidine 0.2% | none | Cremophor RH 40 | 0.006% | 0.003% |
| | | | | | | |
| 2.19 | PHMB 0.0001% | L-Histidine 0.2% | glycerin 3% | Cremophor RH 40 | | |
| 2.94 | PIIMB 0.0001% | L-Histidine 0.2% | glycerin 3% | Cremophor RH 40 | 0.006% | 0.003% |
| | | | | | | |
| 2.19 | PHMB 0.0001% | L-Histidine 0.2% | propylene glycol 3% | Cremophor RH 40 | | |
| 2.95 | PHMB 0.0001% | L-Histidine 0.2% | propylene glycol 3% | Cremophor RH 40 | 0.006% | 0.003% |
| | | | | | | |
| 3.36 | PHMB 0.0001% | L-Histidine 0.2% | sorbitol 5% | Cremophor RH 40 | | |
| 3.92 | PHMB 0.0001% | L-Histidine 0.2% | sorbitol 5% | Cremophor RH 40 | 0.006% | 0.003% |
| 0.68 | Marketed Product 1 | | | | | |
| 2.99 | Marketed Product 2 | | | | | |
| 2.98 | Marketed Product 3 | | | | | |

| | | | | | | |
|---|---|---|---|---|---|---|
| * marketed product 1 having the general composition: A sterile isotonic aqueous solution containing sodium chloride, polyoxyethylene polyoxypropylene block copolymer, sodium phosphate dibasic, sodium phosphate monobasic, and preserved with edetate disodium dihydrate 0.025% and polyhexanide 0.0001 %. ** marketed product 2 having the general composition: A sterile, isotonic solution that contains HYDRANATE (hydroxyalkylphosphonate), boric acid, edetate disodium, poloxamine, sodium borate and sodium chloride; preserved with DYMED (polyaminopropyl biquanide) 0.0001%. | | | | | | |

The data shows that the addition of 0.006% hydrogen peroxide to histidine provides increased antifungal ctivity against *C. albicans.* Consistent results were found in the presence of Cremophor RH40 with glycerin, propylene glycol, and soribitol. All formulations with dilute hydrogen peroxide added to hisitidine were equal to or superior to marketed products.

## Claims

1. An ophthalmic solution, comprising:
0.01 to 1.0 percent by weight L-histidine;
0.0001 to 0.01 percent by weight hydrogen peroxide; and
0.1 to 500 parts per million of a cationic polymeric preservative.

2. The ophthalmic solution of claim 1, further comprising a surface-active agent.

3. The ophthalmic solution of claim 2, wherein said surface-active agent is a hyrdoxy-ethoxylated castor oil.

4. The ophthalmic solution of any of the preceding claims, wherein said cationic polymeric preservative includes polymeric biguanides.

5. The ophthalmic solution of any of the preceding claims, wherein said cationic polymeric preservative includes polymeric hexamethylene biguanides.

6. The ophthalmic solution of claim 4 or 5, wherein said polymeric biguanides have number average molecular weights of at least 1,000.

7. The ophthalmic solution of claim 6, wherein said polymeric biguanides have number average molecular weights from 1,000 to 50,000.

8. The ophthalmic solution of claim 4, wherein said polymeric biguanides are used in combination with a bis(biguanide) compound.

9. The ophthalmic solution of claim 8, wherein said bis(biguanide) compound is alexidine.

10. The ophthalmic solution of any of the preceding claims comprising an optional non-biguanide germicide as a solution preservative.

11. The ophthalmic solution of claim 10, wherein said germicide is present in a concentration of 0.00001 to 0.5 weight percent.

12. The ophthalmic solution of claim 10 or 11, wherein said germicide is present in a concentration of 0.0001 to 0.1 weight percent.

13. The ophthalmic solution of any of claims 10 to 12, wherein said germicide is selected from quaternary ammonium compounds or polymers, thimerosal or other phenylmercuric salts, sorbic acid, alkyl triethalnolamines, and mixtures thereof.

14. The ophthalmic solution of any of the preceding claims, further comprising a buffer.

15. The ophthalmic solution of claim 14, wherein said buffer is selected from boric acid, sodium borate, potassium citrate, citric acid, sodium bicarbonate, bis-tris propane, TRIS and various mixed phosphate buffers, including combination of Na2HPO₄, NaH₂PO₄ and KH₂PO₄, and mixtures thereof.

16. The ophthalmic solution of claim 14 or 15, wherein said buffer is present in amounts ranging from 0.05 to 2.5 percent by weight.

17. The ophthalmic solution of claim 16, wherein said buffer is present in amounts ranging from 0.1 to 1.5 percent by weight.

18. The ophthalmic solution of any of the preceding claims, further comprising other additives, including tonicity agents, demucents, wetting agents, sequestering or chelating agents, enzymes, surfactants and viscosity inducing agents.

19. The ophthalmic solution of claim 18, wherein said ophthalmic solution comprises a chelating agent selected from:
amino carboxylic acid compounds or water-soluble salts thereof, including ethylenediaminetetraacetic acid, nitrilotriacetic acid, diethylenetriaminepentaacetic acid, hydroxyethylethylenediaminetriacetic acid, 1,2-diaminocyclohexanetetraacetic acid, ethylene glycol bis(betaaminoethyl ether) in N, N, N', N' tetraacetic acid (EGTA), aminodiacetic acid and hydroxyethylaminodiacetic acid, citrates, including citric acid and its mono-, di-, and tri-alkaline metal salts;
polyphosphates, including pyrophosphates, triphosphates, tetraphosphates, trimetaphosphates, tetrametaphosphates, as well as more highly condensed phosphates in the form of the neutral or acidic alkali metal salts such as the sodium and potassium salts as well as the ammonium salt.

20. The ophthalmic solution of claim 19, wherein said chelating agent is selected from the di-, tri- and tetra-sodium salts of ethylenediaminetetraacetic acid (EDTA).

21. The ophthalmic solution of claim 19, wherein said chelating agent is disodium EDTA (Disodium Edetate).

22. The ophthalmic solution of claim 18, wherein said tonicity agents include sodium chloride, potassium chloride, glycerol, or mixtures thereof.

23. The ophthalmic solution of claim 22, containing 0.01 to 0.35 weight percent sodium chloride.

24. The ophthalmic solution of claim 18, wherein said surfactants include a polyoxyethylene-polyoxypropylene non-ionic surfactant.

25. The ophthalmic solution of claim 24, wherein said surfactant is selected from poloxamine or poloxamer, wherein the poloxamine surfactant consists of a poly(oxypropylene)-poly(oxyethylene) adduct of ethylene diamine having a molecular weight from 7,500 to 27,000, wherein at least 40 weight percent of said adduct is poly(oxyethylene), and the poloxamer surfactant comprises polyoxyethylene, polyoxypropylene block polymers.

26. The ophthalmic solution of claim 25, comprising the poloxamine surfactant in amounts from 0.01 to 15 weight percent.

27. The ophthalmic solution of any of the preceding claims, further comprising a non-ionic surfactant selected from polyethylene glycol esters of fatty acids, coconut, polysorbate, polyoxyethylene, and polyoxypropylene ethers of higher alkanes (C₁₂-C₁₈).

## Patentansprüche

1. Ophthalmische Lösung, die Folgendes aufweist:
0,01 bis 1,0 Gewichts-% L-Histidin;
0,0001 bis 0,01 Gewichts-% Wasserstoffperoxyd; und
0,1 bis 500 ppm eines kationischen polymeren Konservierungsmittels.

2. Ophthalmische Lösung gemäß Anspruch 1, die ferner ein oberflächenaktives Mittel aufweist.

3. Ophthalmische Lösung gemäß Anspruch 2, wobei das oberflächenaktive Mittel ein hydroxy-ethoxyliertes Rizinusöl ist.

4. Ophthalmische Lösung gemäß irgendeinem der vorhergehenden Ansprüche, wobei das kationische polymere Konservierungsmittel polymere Biguanide aufweist.

5. Ophthalmische Lösung gemäß irgendeinem der vorhergehenden Ansprüche, wobei das kationische polymere Konservierungsmittel polymere Hexamethylenbiguanide aufweist.

6. Ophthalmische Lösung gemäß Anspruch 4 oder 5, wobei die polymeren Biguanide ein Zahlenmittel des Molekulargewichtes von mindestens 1000 besitzen.

7. Ophthalmische Lösung gemäß Anspruch 6, wobei die polymeren Biguanide ein Zahlenmittel des Molekulargewichtes von 1000 bis 50,000 besitzen.

8. Ophthalmische Lösung gemäß Anspruch 4, wobei die polymeren Biguanide in Kombination mit einer Bisbiguanid-Verbindung verwendet werden.

9. Ophthalmische Lösung gemäß Anspruch 8, wobei die Bisbiguanid-Verbindung Alexidin ist.

10. Ophthalmische Lösung nach irgendeinem der vorhergehenden Ansprüche, welche ein optionales desinfizierendes bzw. keimtötendes Nicht-Biguanid als ein Lösungskonservierungsmittel aufweist.

11. Ophthalmische Lösung gemäß Anspruch 10, wobei das keimtötende Mittel in einer Konzentration von 0,00001 bis 0,5 Gewichts-% vorhanden ist.

12. Ophthalmische Lösung gemäß Anspruch 10 oder 11, wobei das keimtötende Mittel in einer Konzentration von 0,0001 bis 0,1 Gewichts-% vorhanden ist.

13. Ophthalmische Lösung gemäß einem der Ansprüche 10 bis 12, wobei das keimtötende Mittel aus folgenden Substanzen ausgewählt ist: quaternären Ammoniumverbindungen oder Polymeren, Thimersal oder anderen Phenylquecksibersalzen, Sorbinsäure, Alkyltriethanolaminen und Mischungen daraus.

14. Ophthalmische Lösung nach irgendeinem der vorhergehenden Ansprüche, die ferner einen Puffer aufweist.

15. Ophthalmische Lösung gemäß Anspruch 14, wobei der Puffer ausgewählt ist aus den folgenden Substanzen: Borsäure, Natriumborat, Kaliumzitrat, Zitronensäure, Natriumbicarbonat, Bistrispropan, TRIS und verschiedene gemischte Phosphatpuffer, einschließlich Kombinationen aus Na₂HPO₄, NaH₂PO₄ und KH₂PO₄, und Mischungen daraus.

16. Ophthalmische Lösung gemäß Anspruch 14 oder 15, wobei der Puffer im Bereich von 0,05 bis 2,5 Gewichts-% vorhanden ist.

17. Ophthalmische Lösung gemäß Anspruch 16, wobei der Puffer im Bereich von 0,1 bis 1,5 Gewichts-% vorhanden ist.

18. Ophthalmische Lösung gemäß irgendeinem der vorhergehenden Ansprüche, die ferner andere Additive aufweisen, einschließlich Tonizitätseinstellmittel, Demulzenz, Benetzungsmittel, Sequestrierungs- oder Chelatmittel, Enzyme, Tenside und Viskosität induzierende Mittel umfassen.

19. Ophthalmische Lösung gemäß Anspruch 18, wobei die ophthalmische Lösung ein Chelatmittel aufweist, das ausgewählt ist auf den folgenden Substanzen:
Aminocarboxylsäureverbindungen oder ihre wasserlöslichen Salze, einschließlich Ethylendiamintetraessigsäure, Nitrilotriessigsäure, Diethylentriaminpentaessigsäure, Hydroxyethylendiamintriessigsäure, 1,2-diaminocyclohexantetraessigsäure, Ethylenglykol-bis(betaaminoethylenether) in N, N, N', N-tetraessigsäure (EGTA), Aminodiessigsäure und Hydroxyethylaminodiessigsäure, Zitraten einschließlich Zitronensäure und ihre Mono-, Di- und Trialkalimetallsalze;
Polyphosphate, einschließlich Pyrophosphaten, Triphosphaten, Tetraphosphaten, Trimetaphosphaten, Tetrametaphosphaten, ebenso wie höher kondensierte Phosphate in Form der neutralen oder sauren Alkalimetallsalze, wie beispielsweise Natrium- und Kaliumsalze ebenso wie Ammoniumsalze.

20. Ophthalmische Lösung gemäß Anspruch 19, wobei der Chelatbildner ausgewählt ist aus den Di-, Tri- und Tetranatriumsalzen der Ethylendiamintetraessigsäure (EDTA).

21. Ophthalmische Lösung gemäß Anspruch 19, wobei der Chelatbildner Dinatrium EDTA (Disodium Edetate) ist.

22. Ophthalmische Lösung gemäß Anspruch 18, wobei die Tonizitätseinstellmittel Natriumchlorid, Kaliumchlorid, Glycerol und Mischungen daraus aufweisen.

23. Ophthalmische Lösung gemäß Anspruch 22, die 0,01 bis 0,35 Gewichts-% Natriumchlorid enthält.

24. Ophthalmische Lösung gemäß Anspruch 18, wobei die Tenside ein nicht-ionisches Polyoxyethylen-Polyoxypropylen-Tensid aufweisen.

25. Ophthalmische Lösung gemäß Anspruch 24, wobei das Tensid ausgewählt ist aus den folgenden Substanzen: Poloxamin oder Poloxamer, wobei das Poloxamin-Tensid aus einem Poly(oxypropylen)-poly(oxyethylen)-Addukt des Ethylendiamins besteht mit einem Molekulargewicht von 7500 bis 27000, wobei mindestens 40 Gewichts-% des Addukts aus Poly(oxyethylen) besteht und das Poloxamer-Tensid Polyoxyethylen und Polyoxypropylenblockpolymere aufweist.

26. Ophthalmische Lösung gemäß Anspruch 25, die Poloxamin-Tensid in Mengen von 0,01 bis 15 Gewichts-% aufweist.

27. Ophthalmische Lösung gemäß irgendeinem der vorhergehenden Ansprüche, die ferner ein nicht-ionisches Tensid aufweist, welches ausgewählt ist aus den folgenden Substanzen: Polyethylenglykolester von Fettsäuren, Kokosnuss, Polysorbat, Polyoxyethylen und aus Polyoxypropylenethern höherer Alkane (C₁₂-C₁₈).

## Revendications

1. Solution ophtalmique, comprenant :
de 0,01 à 1,0 pourcent en poids de L-histidine ;
de 0,0001 à 0,01 pourcent en poids de peroxyde d'hydrogène ; et
de 0,1 à 500 parties par million d'un conservateur polymère cationique.

2. Solution ophtalmique selon la revendication 1, comprenant en outre un agent actif de surface.

3. Solution ophtalmique selon la revendication 2, dans laquelle l'agent actif de surface est une huile de castor hydroxy-éthoxylée.

4. Solution ophtalmique selon l'une quelconque des revendications précédentes, dans laquelle le conservateur polymère cationique comprend des biguanides polymères.

5. Solution ophtalmique selon l'une quelconque des revendications précédentes, dans laquelle le conservateur polymère cationique comprend des biguanides hexaméthylène polymères.

6. Solution ophtalmique selon la revendication 4 ou 5, dans laquelle les biguanides polymères ont des poids moléculaires moyens au moins égaux à 1000.

7. Solution ophtalmique selon la revendication 6, dans laquelle les biguanides polymères ont des poids moléculaires moyens compris entre 1000 et 50000.

8. Solution ophtalmique selon la revendication 4, dans laquelle les biguanides polymères sont utilisés en combinaison avec un composé bis(biguanide).

9. Solution ophtalmique selon la revendication 8, dans laquelle le composé bis(biguanide) est de l'alexidine.

10. Solution ophtalmique selon l'une quelconque des revendications précédentes, comprenant un germicide non biguanide optionnel en tant que solution de conservation.

11. Solution ophtalmique selon la revendication 10, dans laquelle le germicide est présent avec une concentration de 0,00001 à 0,5 pourcent en poids.

12. Solution ophtalmique selon la revendication 10 ou 11, dans laquelle le germicide est présent avec une concentration de 0,0001 à 0,1 pourcent en poids.

13. Solution ophtalmique selon l'une quelconque des revendications 10 à 12, dans laquelle le germicide est choisi parmi des composés ou des polymères quaternaires d'ammonium, du thiomersal ou d'autres sels phénylmercure, l'acide sorbique, des alkyl triéthalnolamines, et leurs mélanges.

14. Solution ophtalmique selon l'une quelconque des revendications précédentes, comprenant en outre une solution tampon.

15. Solution ophtalmique selon la revendication 14, dans laquelle la solution tampon est choisie parmi l'acide borique, le borate de sodium, le citrate de potassium, l'acide citrique, le bicarbonate de sodium, le bis-tris propane, le TRIS et diverses solutions tampon de phosphate mélangées, comprenant une combinaison de Na₂HPO₄, NaH₂PO₄ et de KH₂PO₄, et leurs mélanges.

16. Solution ophtalmique selon la revendication 14 ou 15, dans laquelle la solution tampon est présente dans une quantité comprise entre 0,05 et 2,5 pourcent en poids.

17. Solution ophtalmique selon la revendication 16, dans laquelle la solution tampon est présente dans une quantité comprise entre 0,1 et 1,5 pourcent en poids.

18. Solution ophtalmique selon l'une quelconque des revendications précédentes, comprenant en outre d'autres additifs, comprenant des agents de tonicité, des agents adoucissants, des agents mouillants, des agents séquestrants ou des agents chélateurs, des enzymes, des agents de surface et des agents inducteurs de viscosité.

19. Solution ophtalmique selon la revendication 18, dans laquelle la solution ophtalmique comprend un agent chélateur choisi parmi :
des composés d'acide aminocarboxylique ou leurs sels solubles dans l'eau, comprenant l'acide éthylènediaminetétraacétique, l'acide nitrilotriacétique, l'acide diéthylène-triaminepentaacétique, l'acide hydroxyéthyléthylènediamine-triacétique, l'acide 1,2-diaminocyclohexanetétraacétique, le bis(bétaaminoéthyle éther) d'éthylène glycol dans de l'acide N, N, N', N' tétraacétique (EGTA), l'acide aminodiacétique et l'acide hydroxyéthylaminodiacétique,
des citrates, comprenant l'acide citrique et ses sels métalliques mono-, di-, et tri-alkalins ;
des polyphosphates, comprenant les pyrophosphates, les triphosphates, les tétraphosphates, les trimétaphosphates, les tétramétaphosphates, ainsi que des phosphates plus fortement condensés sous forme de sels métalliques alcalins neutres ou acides tels que les sels de sodium et de potassium ainsi que le sel d'ammonium.

20. Solution ophtalmique selon la revendication 19, dans laquelle l'agent chélateur est choisi parmi les sels di-, tri- et tétra-sodiques d'acide éthylènediaminetétraacétique (EDTA).

21. Solution ophtalmique selon la revendication 19, dans laquelle l'agent chélateur est de l'EDTA disodique (Disodium Edetate).

22. Solution ophtalmique selon la revendication 18, dans laquelle les agents de tonicité comprennent le chlorure de sodium, le chlorure de potassium, le glycérol, ou leurs mélanges.

23. Solution ophtalmique selon la revendication 22, contenant de 0,01 à 0,35 pourcent en poids de chlorure de sodium.

24. Solution ophtalmique selon la revendication 18, dans laquelle les agents de surface comprennent un agent de surface non ionique polyoxyéthylène-polyoxypropylène.

25. Solution ophtalmique selon la revendication 24, dans laquelle l'agent de surface est choisi du type poloxamine ou poloxamère, dans laquelle l'agent de surface poloxamine est constitué d'un adjuvent poly(oxypropylène)-poly(oxyéthylène) d'éthylène diamine ayant un poids moléculaire compris entre 7500 et 27000, au moins 40 pourcent en poids dudit adjuvent sont du poly(oxyéthylène), et l'agent de surface poloxamère comprend des polymères blocs poly-oxyéthylène, poly-oxypropylène.

26. Solution ophtalmique selon la revendication 25, comprenant l'agent de surface poloxamine dans des quantités comprises entre 0,01 et 15 pourcent en poids.

27. Solution ophtalmique selon l'une quelconque des revendications précédentes, comprenant en outre un agent de surface non ionique choisi parmi des esters polyéthylèneglycol d'acides gras, de noix de coco, le polysorbate, des éthers polyoxyéthylène ou polyoxypropylène d'alkanes supérieurs (C₁₂-C₁₈) .
